(19) Europäisches Patentamt / European Patent Office / Office européen des brevets

(11) **EP 2 347 242 B1**

(12) **EUROPEAN PATENT SPECIFICATION**

(45) Date of publication and mention
of the grant of the patent:
**23.09.2015 Bulletin 2015/39**

(21) Application number: **09740704.3**

(22) Date of filing: **28.10.2009**

(51) Int Cl.:
*G01N 13/00* (2006.01)    *G01N 33/15* (2006.01)

(86) International application number:
**PCT/EP2009/064212**

(87) International publication number:
**WO 2010/049447 (06.05.2010 Gazette 2010/18)**

(54) **TWO-COMPARTMENT DISSOLUTION APPARATUS AND PROCESS**

AUFLÖSUNGSVORRICHTUNG MIT ZWEI FÄCHERN UND VERFAHREN

APPAREIL ET PROCÉDÉ DE DISSOLUTION À DEUX COMPARTIMENTS

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **28.10.2008 EP 08167702**

(43) Date of publication of application:
**27.07.2011 Bulletin 2011/30**

(73) Proprietor: **LEK Pharmaceuticals d.d.
1526 Ljubljana (SI)**

(72) Inventors:
• **LEGEN, Igor**
**1526 Ljubljana (SI)**
• **KOCEVAR, Klemen**
**1526 Ljubljana (SI)**

(74) Representative: **Prüfer & Partner GbR
European Patent Attorneys
Sohnckestraße 12
81479 München (DE)**

(56) References cited:
**WO-A-99/28437    WO-A-2008/112245**

• **G. CORTI ET AL: EUROPEAN JOURNAL OF
PHARMACEUTICAL SCIENCES, vol. 27, 15
December 2005 (2005-12-15), pages 346-353,
XP002524480**
• **HUQUE F T T ET AL: "Permeability through DOPC/
dodecane membranes: measurement and LFER
modelling" EUROPEAN JOURNAL OF
PHARMACEUTICAL SCIENCES, ELSEVIER,
AMSTERDAM, NL, vol. 23, no. 3, 1 November 2004
(2004-11-01), pages 223-232, XP004601706 ISSN:
0928-0987**

**Description**

The present invention relates to an apparatus for testing dissolution of an active pharmaceutical ingredient. Furthermore, the present invention relates to a process for testing dissolution of an active pharmaceutical ingredient and a process for assaying or predicting in vivo dissolution of an active pharmaceutical ingredient.

Generally, a dissolution test for an active pharmaceutical ingredient (API) measures the rate of release of the drug by determining the amount of API that goes into solution as a function of time. For example, a dissolution of an API from a solid dosage form involves at least two consecutive steps. A first step is liberation of the API from the matrix of a pharmaceutical composition, the so called "disintegration". In a second step, dissolution of the API in the liquid medium occurs, that is the solubilisation of the API particles. If the disintegration is the rate-limiting step of dissolution, the rate of dissolution is considered to be disintegration controlled. On the other hand, if the solubilisation of the API in the liquid medium is the rate-limiting step of dissolution, the rate of dissolution is intrinsic dissolution controlled. Dissolution testing of poorly soluble APIs is an important issue in pharmacy to develop and evaluate new pharmaceutical formulations.

Not only a pharmaceutical formulation or preparation as a whole, also that of the conditions and/or forms of an API itself constitute critical factors affecting the pharmacologically relevant dissolution. In order to increase solubilisation of the API and Consequently absorption from the gastrointestinal tract, many different approaches can be used, for example decreasing the particle size thus increasing the surface area, preparing different polymorphic forms, converting crystals into the amorphous state, adding surfactants into the formulations, and so on. As a result, many different formulations are designed in the development phase. In order to avoid extensive pharmacokinetic trials on human volunteers, a development of an *in vitro* method that can reliably and reproducibly predict the differences in the absorption of an API from different formulations would be highly beneficial.

Several concepts for testing dissolution of APIs are known in prior art.

The US Pharmacopeia USP31 describes several different apparatus for performing dissolution testing. Among others, a paddle apparatus and a flow through cell are described for testing dissolution of APIs.

US 2003/0088369 A1 suggests the use of a cascade of three stirred flow-through cells, which is a complex modification of the flow through cell described in USP 31 for predicting *in vivo* performance of poorly soluble APIs.

E. Nicolaides et al., Pharmaceutical Research, Vol. 16, No. 12, 1999, p. 1876-1882 and J.B. Dressman et al., European Journal of Pharmaceutical Sciences 11 Suppl. 2 (2000), p. 73-80 describe the use of *in vivo* relevant media (FaSSIF: fasted state small intestine and FeSSIF: fed state small intestine) together with a paddle apparatus described in USP 31 for in vitro-*in vivo* correlations of lipophilic, poorly water-soluble APIs.

V.H. Sunesen et al., European Journal of Pharmaceutical Sciences 24 (2005), p. 305-313 describe the use of modified fasted state/small intestine (FaSSIF) and fed state/small intestine (FeSSIF) together with the flow though cell described in USP 31 to establish an *in vitro-in vivo* correlation for poorly soluble APIs.

WO 2008/112245 discloses a device and a method for determining the release profile of a macromolecule from a pharmaceutical composition comprising the macromolecule. The device comprises two chambers separated by a semi-permeable barrier which is adapted to prevent an undissolved macromolecule from passing from the first chamber to the second chamber. This arrangement provides for a fluid communication wherein a fluid added to either of the first chamber or the second chamber is able to flow between the chambers.

WO 99/28437 discloses a system and a method for assessing simulated biological dissolution of a pharmaceutical formulation and absorption of a pharmaceutically active compound thereof. In this system, an apical compartment and a basal compartment are separated by a filter containing a monolayer of cells, wherein the filter is capable of supporting the cell monolayer.

However, when it comes to dissolution testing of poorly soluble APIs, the above indicated prior art apparatus for dissolution testing of APIs frequently fail to properly predict the in vivo performance.

Therefore, according to the object of the present invention, there is a need for an improved concept for testing dissolution of APIs. The object is solved by a system according to claim 1 and a process according to claim 5.

G. Corti et al., "Development and evaluation of an in vitro method for prediction of human drug absorbtion - I. Assessment of artificial membrane composition", European Journal of Pharmaceutical Sciences, vol. 27, 2005, p. 346-353, discloses a method for measuring the amount of drug permeated per unit of time. In this method, the API in the form of naproxen is provided in completely dissolved state, namely in the form of a solution having a concentration being 50 times lower than its saturation solubility.

Huque F.T.T. et al., "Permeability through DOPC/dodecane membranes: measurement and LFER modelling", European Journal of Pharmaceutical Sciences, Elsevier, Amsterdam, vol. 23, no. 3, 2004, p. 223-232, discloses an arrangement wherein a filter coated with 2% (w/v) dioleylphophatidylcholine in dodecane is placed between two composite wells. In this arrangement, the API in the form of an ionisable compound is provided as a 1 mM stock solution, that is, the API is present in completely dissolved state in order to measure its permeability through the coated filter.

Summary of the invention

The present invention provides an apparatus for testing dissolution of an active pharmaceutical ingredient, said apparatus comprising two compartments separated by a membrane impregnated with a lipophilic phase, wherein the membrane is permeable to a dissolved state but impermeable to an undissolved state of said active pharmaceutical ingredient.

The present invention further provides a use of a membrane comprising a polymer material and a lipophilic phase for testing dissolution of an active pharmaceutical ingredient.

Furthermore the present invention provides a process for testing dissolution of an active pharmaceutical ingredient in predetermined condition or form, comprising the steps of:

- providing a chamber comprising two compartments separated by a membrane impregnated with a lipophilic phase, one compartment being a donor compartment donating the active pharmaceutical ingredient and the other compartment being the acceptor compartment accepting or receiving the active pharmaceutical ingredient,
- placing a donor medium comprising at least an active pharmaceutical ingredient and an aqueous solution in the donor compartment,
- placing an acceptor medium in the acceptor compartment, wherein the active pharmaceutical ingredient is allowed to pass the membrane from the donor compartment to the acceptor compartment, and
- measuring and/or detecting the concentration of active pharmaceutical ingredient or a reaction or decomposition product thereof, either in the acceptor medium, or measuring and/or detecting in the donor medium, or both in the acceptor medium and in the donor medium.

The present invention still further provides a process for assaying or predicting in vivo dissolution of an active pharmaceutical ingredient, wherein a permeation of the active pharmaceutical ingredient through a membrane comprising a lipophilic phase is measured and/or determined.

The present invention yet further provides a process for obtaining an active pharmaceutical ingredient with desired dissolution properties, comprising the steps of:

- providing multiple active pharmaceutical ingredients respectively varying in at least one condition or form,
- subjecting each active pharmaceutical ingredient of the predetermined condition or form to a process according to claim 4 or a process according to claim 9, and
- selecting the active pharmaceutical ingredient in predetermined condition or form which displays the desired dissolution properties.

Description of the invention and its preferred embodiments

According to the present invention, it was surprisingly found that realistic physiological conditions can be simulated by simple means, even under conditions wherein an API has a poor solubility. Therefore, an exceptional reliable prediction of in vivo dissolution is provided. Furthermore, additional advantages over prior art are given. For example, an advantage of the concept of the present invention is that only minimum amounts of testing media are required, which provides for significant cost reductions, as much less amounts of expensive in vivo relevant additives and API to be tested are required. Besides, said simple means for simulating intestinal blood flow enable a simple constitution of the testing apparatus providing a simple handling as well as a simple maintenance, which in turn provides for reduced running costs of the testing apparatus.

Various aspects, advantageous features and preferred embodiments of the present invention as summarized in the following items, respectively alone or in combination, contribute to solving the object of the invention:

(1)
A system for testing dissolution of an active pharmaceutical ingredient, which contains an apparatus comprising two compartments separated by a membrane, wherein the membrane is permeable to a dissolved state but impermeable to an undissolved state of said active pharmaceutical ingredient, whereby the system comprises a donor medium placed in the first compartment representing the donor compartment and the system comprises an acceptor medium placed in the second compartment representing the acceptor compartment
characterised in that
said membrane is impregnated with a lipophilic phase and that the active pharmaceutical ingredient is more soluble in the acceptor medium than in the donor medium or that acceptor medium is capable of reacting with the active pharmaceutical ingredient to thereby consume the active pharmaceutical ingredient by chemical reaction, so that a driving force is provided effecting that the active pharmaceutical ingredient passes the membrane from the donor compartment to the acceptor compartment.

The term "dissolution" as used herein refers to dissolution in the intestine comprising the steps of disintegration of an API or a pharmaceutical formulation comprising the API and following solubilisation of the API. Surprisingly it was found that a membrane comprising a lipophilic phase enables a simple and effective means for simulating the continuous wash away of the API by the intestinal blood flow. Thus, the apparatus of the present invention governing dissolution of an API to be tested has a significant prerequired step of diffusion of the dissolved API across said membrane, which is - though preferably being artificial - lipophilic for realistically simulating the situation in the intestine.

The impregnation of the membrane with said lipophilic phase provides for a suitable mimics of physiological membranes relevant for dissolution of APIs, in particular of the intestinal epithelial membrane.

(2) The system according to item (1), comprising a first compartment representing a donor compartment and a second compartment representing an acceptor compartment, wherein the apparatus is arranged such that when a donor medium comprising active pharmaceutical ingredient in undissolved state and optionally also in dissolved state is placed in said first compartment representing the donor compartment, said second compartment representing the acceptor compartment comprises only active pharmaceutical ingredient in dissolved state in an acceptor medium.

(3) The system according to item (1) or (2), wherein means for measuring and/or detecting the concentration of active pharmaceutical ingredient or a reaction or decomposition product thereof, is (are) arranged either in the acceptor compartment, or in the donor compartment, or both in the acceptor compartment and in the donor compartment.

(4) The system according to any one of items (1) to (2), wherein means for withdrawing sample(s) for subsequent measurement and/or detection of the concentration of active pharmaceutical ingredient or a reaction or decomposition product thereof, is (are) arranged either in the acceptor compartment, or in the donor compartment, or both in the acceptor compartment and in the donor compartment.

(5) The system according to any one of items (1) to (4), wherein the apparatus is arranged such that when a donor medium comprising active pharmaceutical ingredient is placed in said first compartment representing the donor compartment and acceptor medium is placed in said second compartment representing the acceptor compartment, a driving force is provided effecting that the API passes the membrane from the donor compartment to the acceptor compartment.
In this way, an effective driving force can be beneficially provided for simulating the continuous wash away of API by the intestinal blood flow. The reliability of this simulation depends on the appropriate combination of the kind of API, the kind of membrane, the kind of donor medium and the kind of acceptor medium. Effectively, this simulation can be accomplished, for example, by withdrawing API from the acceptor compartment, by decomposing API in the acceptor compartment, by reacting or by binding API in the acceptor medium.

(6) The system according to any one of items (1) to (5), arranged such that fluid of the donor medium present in the donor compartment does substantially or entirely not flow to the acceptor compartment, and fluid of the acceptor medium present in the acceptor compartment substantially or entirely does not flow to the donor compartment; preferably, there is no flow of any fluid through the membrane in either direction.
This measure provides a condition of essential or complete lack of flow of fluid between the donor compartment and the acceptor compartment. This can be suitably controlled according to the invention by a correspondingly adjusted impregnation of the membrane with a lipophilic phase. Since said fluid is generally water or based on water, the membrane being ' impregnated with a lipophilic phase can act as "lipid barrier layer" which provides for simulating a functional membrane under or "close to" physiological conditions. Thereby, it is advantageously achieved that fluid in form of water or a water based solution substantially does not flow through said membrane, preferably flow of fluid through the membrane is even avoided.
The term "fluid" as used herein means a fluid used as liquid component(s) of the acceptor medium and the donor medium independently from each other, wherein said liquid component(s) is preferably a solvent, more preferably said solvent is water and/or liquid compound(s) having any one of lipophobic, hydrophilic and water-miscible characteristics such as C1-C4-alcohols, and in particular said solvent is water.

(7) The system according to any one of items (1) to (6), wherein said membrane prior to impregnation with the lipophilic phase is an artificial membrane.
In this way it is possible to systematically tailor the characteristics of the membrane and to control its quality.

Furthermore, the characteristics of an artificial membrane will not or scarcely deteriorate during storage. Thus, reproducibility is more ensured.

Since the membrane is artificial, it does not require any particular conditions, i.e. pH or any conditions which could be unsuitable if using cultured cells, in the donor or in the acceptor compartment.

(8) The system according to any one of the preceding items, wherein the membrane prior to impregnation with the lipophilic phase has an average pore size in a range of 0.1 $\mu$m to 10 $\mu$m.

This embodiment provides for an appropriate setting and preconditioning of a certain permeation performance of API through the membrane. This may be useful to adjust the system to a certain type of API, and/or to come close to physiological conditions.

(9) The system according to any one of the preceding items, wherein said membrane comprises a polymer selected from the group consisting of cellulose or cellulose derivatives, poly(tetrafluoroethylene), polyvinylidene fluoride, nylon and polycarbonate.

(10) The system according to item (9), wherein said membrane is made of cellulose nitrate or cellulose acetate.

The embodiments defined by items (9) and (10) allows a suitable adaption depending on the nature of the acceptor medium. Since the acceptor medium does not necessarily reflect physiological conditions, the membrane might be exposed to chemically very aggressive media at its surface contacting the acceptor medium. For example, strong inorganic acids or bases, or reactants to react with the API, may be contained in the acceptor medium. As a beneficial option, the membrane can be selected to be resistant to the acceptor medium. As another beneficial option, the membrane mentioned is widely available and, even more preferred, allows to reliably provide prescribed pore sizes.

(11) The system according to any one of the preceding items, wherein the lipophilic phase is formed of a compound selected from the group consisting of hydrocarbons, fatty acids, fatty alcohols and fatty acid esters of polyhydric alcohol.

(12) The system according to item (11), wherein the hydrocarbon is selected from the group consisting of $C_5$-$C_{50}$ n-alkanes such as n-heptadecane, n-octadecane, n-nonadecane, n-eicosane, n-heneicosane, n-docosane, n-tricosane, n-tetracosane, n-pentacosane, n-triacontane, n-pentatriacontane, n-tetracontane and n-pentacontane and mixtures comprising at least two of the aforementioned hydrocarbons, preferably petrolatum, paraffin wax and microcrystalline wax.

(13) The system according to item (11), wherein the fatty acids are selected from the group consisting of butyric acid, caproic acid, caprylic acid, capric acid, lauric acid, myristic acid, palmitic acid, stearic acid, arachidinic acid, behenic acid, lignoceric acid, cerotic acid, oleic acid, myristoleic acid, linoleic and mixtures comprising at least two of the aforementioned fatty acids.

(14) The system according to item (11), wherein the fatty alcohol is selected from the group consisting of capryl alcohol 1-octanol, 2-ethyl hexanol, 1-nonanol, 1-decanol, decyl alcohol, 1-dodecanol, 1-tetradecanol, 1-hexadecanol, cis-9-hexadecen-1-ol, 1-octadecanol, 16-methylheptadecan-1-ol, 9E-octadecen-1-ol, cis-9-octadecen-1-ol, 9Z, 12Z-octadecadien-1-ol, 9E, 12E-octadecadien-1-ol, 9Z, 12Z, 15Z-octadecatrien-1-ol, 9E, 12E, 15-E-octadecatrien-1-ol, 12-hydroxy-9-octadecen-1-ol, 1-eicosanol, 1-docosanol, cis-13-docosen-1-ol, 1-tetracosanol, 1-octacosanol, 1-triacontanol, 1-tetratriacontanol and mixtures comprising at least two of the aforementioned fatty alcohols.

(15) The system according to item (11), wherein the fatty acid esters of polyhydric alcohol are selected from the group consisting of esters formed by esterification of an alcohol having two or more hydroxyl groups in the molecule, preferably alkylene glycols, and polyalkylenglycols, more preferably ethylene glycol, propylene glycol, polyethylene glycol, polypropylene glycol or copolymers of them, glycerol fatty acid esters.

The embodiments according to items (11) to (15) define special selections of compounds which are providing the lipophilic phase in a way which can sufficiently mimic physiological membranes relevant for dissolution of APIs, and particularly suitable for impregnating the membrane with a lipophilic phase. The different compounds belonging to the different groups defined in item (11) can be properly selected depending on the nature of the membrane. The choice of said compounds may also depend on the kind of API and acceptor medium used.

(16) The system according to any one of the preceding items, wherein means for mixing a medium in the compartments are provided.

(17) The system according to item (16), wherein the means for mixing comprises one or more apertures for supplying gas to a medium contained in the compartments.

(18) The system according to item (17), wherein the gas comprises one, two or more gaseous elements, preferably the gas is a mixture of carbon dioxide and oxygen.

(19) The system according to any one of the preceding items, wherein means for thermostating the compartments are provided.

The embodiments defined by items (16) to (19) provide for a more realistic simulation of physiological conditions in view of circulation of body fluids, gases contained in said body fluids and temperature conditions within a body. Thus, these means further contribute to an improved correlation between in vivo and in vitro dissolution.

(20) Use of a membrane comprising a polymer material and a lipophilic phase for testing dissolution of an active pharmaceutical ingredient.
As already elucidated above, the membrane provides for a realistic simulation of an intestinal membrane. In particular, an artificial membrane is preferred which enables to exactly define the pore size of the membrane.

(21) Use according to item (20), wherein the membrane is as defined in any one of items (7) to (14), or wherein the membrane is used for an apparatus according to any one of items (1) and (16) to (19).

(22) Process for testing dissolution of an active pharmaceutical ingredient, comprising the steps of:

- providing a chamber comprising two compartments separated by a membrane impregnated with a lipophilic phase, one compartment being a donor compartment donating the active pharmaceutical ingredient, and the other compartment being the acceptor compartment accepting or receiving the active pharmaceutical ingredient,
- placing a donor medium comprising at least an active pharmaceutical ingredient and an aqueous solution in the donor compartment,
- placing an acceptor medium in the acceptor compartment, wherein the active pharmaceutical ingredient is allowed to pass the membrane from the donor compartment to the acceptor compartment, and
- measuring and/or detecting the concentration of active pharmaceutical ingredient or a reaction or decomposition product thereof, either in the acceptor medium, or measuring and/or detecting in the donor medium, or both in the acceptor medium and in the donor medium,

wherein a driving force between the donor compartment and the acceptor compartment is provided by selecting an acceptor medium in which the active pharmaceutical ingredient is more soluble than in the donor medium, or wherein the active pharmaceutical ingredient is consumed by chemical reaction.

In such a process, a donor medium donating the API and an acceptor medium accepting the API are used. Donating API means that API is at least partly dissolved in the donor medium. The donor medium may preferably reflect conditions within the intestine by appropriately adjusting its composition by appropriately adjusting composition, e.g. by selecting appropriate compounds such as buffers, bile salts, phospholipids, fatty acids and/or monoglycerides, and status, e.g. pH, pH-buffer capacity, concentration of the different ingredients, consistency, viscosity. The composition of the donor medium may be selected in view of the conditions of the intestine to be simulated. For example, the composition of a donor medium for simulating fasted state in the small intestine (FaSSIF) will differ from that simulating fed state in the small intestine (FeSSIF). The acceptor medium may not necessarily reflect physiological conditions, and it is preferred that it does not so. Rather it is particularly preferred that the solubility of the API tested is higher in the acceptor medium compared to the donor medium, more preferably much higher. "Higher/much higher" used herein means 2 times, preferable 5 times, more preferably 10 times and in particular 50 times higher. Provision of acceptor medium allows to set conditions independent from donor medium. According to a preferred embodiment, an effective driving force can be beneficially provided for simulating the continuous wash away of API by the intestinal blood flow. Said driving force effects that the API passes the membrane from the donor compartment to the acceptor compartment. The reliability of this simulation depends on the appropriate combination of the kind of API, the kind of membrane, the kind of donor medium and the kind of acceptor medium. The amount of API which passed the membrane, or a reaction or decomposition product thereof, can then be measured in the acceptor medium, preferably within (a) predetermined time interval(s). Depending on the nature of API, the concentration of active pharmaceutical ingredient or a reaction or decomposition product thereof is measured and/or detected either in the acceptor medium, or measured and/or detected in the donor medium, or both in the acceptor medium and in the donor medium. The amount of API measured within predetermined time interval(s) may then be compared with *in vivo* pharmacokinetic data. Surprisingly, the process of the present invention provides for reliable in vivo-in vitro correlation(s) by relatively simple means.

(23) The process according to item (22), wherein said donor compartment, said acceptor compartment and said membrane impregnated with a lipophilic phase are arranged such that a fluid placed either to the donor or to the acceptor compartment is substantially not able to flow through the membrane impregnated with a lipophilic phase, preferably there is no flow of fluid through the membrane impregnated with a lipophilic phase.
Further reference is made to the explanation in item (6) above.
The term "fluid" as used herein means a fluid used as liquid component(s) of the acceptor medium and the donor medium independently from each other, wherein said liquid component(s) is preferably a solvent, more preferably said solvent is water and/or liquid compound(s) having any one of lipophobic, hydrophilic and water-miscible characteristics such as C1-C4-alcohols, and in particular said solvent is water. In this way, it is effectively avoided that fluid of the donor and/or acceptor medium flows from the donor compartment to the acceptor compartment and vice versa. Since the membrane is impregnated with a lipophilic phase, flow through of lipophobic fluid such as water is effectively suppressed. This provides for a particularly exact dissolution testing.

(24) The process according to item (22) or (23), wherein said aqueous solution is a buffer solution.
This embodiment further provides for realistic physiological conditions, since body fluids such as blood are buffered, too.

(25) The process according to any one of items (22) to (24), wherein the membrane impregnated with a lipophilic phase is exposed to said aqueous solution for a predetermined time.

(26) The process according to item (25), wherein the predetermined time is in a range of 0.2 to 2 h, preferably 0.5 to 1.5 h and more preferably about 1 h.
The measures defined in items (25) and (26) ensure that the lipid phase is saturated with aqueous phase and thus provides for reliable, repeatable and realistic test conditions.

(27) The process according to any one of items (22) to (26), wherein the active pharmaceutical ingredient or a pharmaceutical composition comprising the active pharmaceutical ingredient is placed into the donor compartment in a solid or dispersed form. In this way, it is possible to test dissolution behaviour of an API alone as well as that of a pharmaceutical composition comprising the API. For a new, hardly investigated API, first dissolution tests may be carried out with the API alone in order to collect first dissolution data. Using a pharmaceutical composition comprising the API allows to optimize the dissolution behaviour of the pharmaceutical composition by varying drug load, type and amount of excipient, compression degree, type of processing and so on.

(28) The process according to any one of items (22) to (27), wherein a concentration gradient between the donor compartment and the acceptor compartment is provided by selecting an acceptor medium in which the active pharmaceutical ingredient is more soluble than in the donor medium, or wherein the active pharmaceutical ingredient is consumed by chemical reaction.
This measure provides the driving force for an API to permeate through the membrane. It is understood that the selection of the dissolution medium strongly depends on the nature of the API to be tested. Since the membrane is impregnated with a lipophilic phase, it is made possible that substantially or entirely no and preferably no fluid flows through the membrane. This provides for a particularly exact dissolution testing.

(29) The process according to any one of items (22) to (28), wherein the donor medium and the acceptor medium is mixed by mixing means.

(30) The process according to any one of items (22) to (29), wherein the donor medium and the acceptor medium is thermostasized.

(31) The process according to item (30), wherein thermostating is at physiological temperatures of a mammal, preferably between 25 and 42°C, more preferably between 35 to 40°C and most preferably about 37°C.
As to this embodiment of the process and its significance, reference is made to the explanations for items (16) to (19) above.

(32) The process according to any one of items (22) to (31),

wherein one or more sample volume(s) of the acceptor medium in the acceptor compartment are taken every predetermined time interval(s), and said one or more sample volume(s) is (are) replaced by fresh acceptor medium after taking said sample volume(s).

(33) The process according to item (32), wherein the sample volumes are analytically analyzed for the amount of active pharmaceutical ingredient comprised in each sample volume.

(34) The process according to any one of items (22) to (33),
wherein two or more varying conditions or forms of active pharmaceutical ingredient are separately processed and measured/detected in order to compare the amount of each of said active pharmaceutical ingredient in varying conditions or forms or a reaction or decomposition product thereof contained in the acceptor medium at a predetermined time interval.

The embodiments defined in items (32) to (34) provide measures for an accurate measurement procedure.

(35) The process according to any one of items (22) to (34), wherein the buffer used for the aqueous buffer solution is a Ringer buffer.
This measure is particularly suited for simulating physiological buffer systems.

(36) The process according to any one of items (22) to (35), wherein the acceptor medium is an aqueous solution of an acid or base, preferably an inorganic acid or base.
As elucidated under item (28), the nature of the dissolution media strongly depends on the nature of the API. According to this embodiment, the API shows a considerably improved solubility in an acid or base compared to neutral medium.

(37) The process according to any one of items (22) to (36), wherein the active pharmaceutical ingredient has a solubility of less than 100 $\mu$g/ml in an aqueous medium having a pH of about 7.
In this way, the present process provides reliable correlation between in vivo and in vitro dissolution even with compounds having very low solubility.

(38) The process according to any one of items (22) to (37), wherein the pH of the donor medium is in a range of about pH 6.0 to 7.5.

(39) The process according to any one of items (22) to (38), wherein the aqueous solution comprises bile salts.

(40) The process according to item (39), wherein the bile salts are present in a concentration range of 0.1 to 50 mM.

(41) The process according to any one of items (36) to (40), wherein the aqueous buffer solution comprises phospholipids, preferably lecithin.

(42) The process according to item (41), wherein the phospholipids are present in a concentration range of 0.1 to 10 mM.

(43) The process according to any one of items (22) to (42), wherein the membrane prior to impregnation with the lipophilic phase has an average pore size in a range of 0.1 $\mu$m to 10 $\mu$m.

The embodiments defined by items (38) to (43) particularly reflect the physiological conditions in the intestine.

(44) A process for assaying or predicting in vivo dissolution of an active pharmaceutical ingredient, wherein a permeation of the active pharmaceutical ingredient through a membrane comprising a lipophilic phase is measured and/or determined.
According to this aspect of the invention, it is possible to provide for realistic physiological conditions resulting in reliable in vivo-in vitro correlations.

(45) A process for obtaining an active pharmaceutical ingredient with desired dissolution properties, comprising the steps of:

-   providing multiple active pharmaceutical ingredients respectively varying in at least one condition or form,

- subjecting each active pharmaceutical ingredient of the predetermined condition or form to a process according to item (22) or a process according to item (44), and
- selecting the active pharmaceutical ingredient in predetermined condition or form which displays the desired dissolution properties.

According to this further aspect of the invention, a reliably screening for an active pharmaceutical ingredient in predetermined condition or form having the desired dissolution properties is provided. Thereby, the number of extensive and costly *in vivo* dissolution tests in order to find the pharmaceutical composition having the desired dissolution properties can be significantly reduced. Furthermore, the process provides for an effective

(46) The process according to items (44) or (45), using an apparatus according to any one of items (1) and (16) to (19).

(47) The process according to any one of items (44) to (46), wherein the membrane is as defined in any one of items (7) to (14), or wherein the process is performed by a process according to any one of items (21) to (43).

(48) The process according to any one of items (44) to (47), wherein the condition of the active pharmaceutical ingredient is selected from particle size, polymorphic forms, bulk density and type of processing.

(49) The process according to any one of items (44) to (48), wherein the active pharmaceutical ingredient is incorporated into a pharmaceutical composition varying in drug load, type and amount of excipient, compression degree and type of processing.

(50) A system for testing dissolution of an active pharmaceutical ingredient, which comprises an apparatus according any one of items (1) to (19), wherein a donor medium comprising at least an active pharmaceutical ingredient in undissolved state and an aqueous solution is placed in one compartment representing a donor compartment, and an acceptor medium is placed in the other compartment representing an acceptor compartment, wherein active pharmaceutical ingredient in a dissolved state is allowed to pass the membrane from the donor compartment to the acceptor compartment.

The embodiments defined in items (48), (49) and (50) provide measures for testing the dissolution behaviour of an API alone or an API incorporated into a pharmaceutical formulation. While first dissolution tests may be carried out with the API alone in order to study dissolution of the influence of particle size, polymorphic forms, bulk density on its dissolution behaviour, further tests may be carried out with pharmaceutical compositions comprising the API in order to optimize its formulation in view of dissolution of API.
For the API alone as well as for the pharmaceutical composition comprising the API, the type of processing may have an impact on the dissolution behaviour. For example, particles may be manufactured by different types of processing such as dry or wet granulation or by precipitation.

Detailed description of the invention

The present invention is now described in more detail by referring to further preferred and further advantageous embodiments and examples, which are however presented for illustrative purpose only and shall not be understood as limiting the scope of the present invention.
In order to improve a concept for testing dissolution of an API, extensive test series were carried out by the inventors to find critical factors that are particularly suited to reliably predict in vivo dissolution.
Conventionally, dissolution tests of APIs are conducted in dissolution apparatus having one compartment for a test medium, e.g. the dissolution apparatus described in the US
Pharamcopeia. However, in such a "one compartment apparatus", dissolution of the tested API is governed by the difference between the solubility of the tested API and the concentration of the dissolved API in the same dissolution medium.
The US Pharmacopeia especially encouraged sink conditions for dissolution testing. "Sink condition" describes a dissolution system wherein the concentration of API is sufficiently dilute so that the dissolution process is not impeded by approach to saturation of the compound of interest, that is the API. Typically, sink conditions are considered to exist if the volume of dissolution media is equal to or greater than three times necessary to make a saturated solution of the API. Under a sink condition, dissolution of the API is not limited by, or determined by the solubility in the dissolution medium. However, for poorly soluble APIs, sink conditions do not occur *in vivo*. The concentration of API in the intestinal tract will reach saturation, and the dissolution rate will then depend on the rate of absorption of the active substance across the intestinal epithelium into the intestinal blood vessels.

Therefore, in a one compartment dissolution apparatus, e.g. according to the US Pharmacopeiea USP 31, the dissolution rate of the API will depend mainly on the composition of the dissolution medium. The solubility of poorly soluble APIs in *in vivo* relevant dissolution media, such as those described in corresponding documents (e.g. E. Nicolaides et al., Pharmaceutical Research, Vol. 16, No. 12, 1999, p. 1876-1882; J.B. Dressman et al., European Journal of Pharmaceutical Sciences 11 Suppl. 2 (2000), p. 73-80; V.H. Sunesen et al., European Journal of Pharmaceutical Sciences 24 (2005), p. 305-313) is very often too low to obtain useful dissolution data that can be correlated to *in vivo* data. The solubility of the tested API can be increased by the addition of higher concentrations of surfactants or by the addition of organic solvents. However, in this case, the dissolution medium does not reflect the *in vivo* conditions in the intestinal tract, which very often leads to poor *in vivo* predictability. The flow through cell described in US Pharmacopeia (USP 31) and the cascade of stirred flow through cells described in US 2003/0088369 A1 may to a certain extent overcome the saturation constraint when using *in vivo* relevant dissolution medium, because the concentration of dissolved API is maintained low by constant application of fresh dissolution medium. However, this conventional approach can not reliably simulate *in vivo* conditions. *In vivo*, the dissolution is further determined by the absorption across the lipophilic cell membranes of the gut walls. In contrast to in vivo conditions, in case of the flow though cell according to USP 31 and the cascade of flow through cells according to US 2003/0088369 A1, dissolution is controlled by the rate of addition of fresh medium and by the rate of removing of medium wherein the API is dissolved. Therefore, those conventional flow through cells strongly depend on such unphysiological parameters. Furthermore, the above mentioned flow-through cells require vast amounts, that is at least 100 ml, of test medium for carrying out one dissolution test.

On the other hand, the concept according to the present invention enables the use of *in vivo* relevant dissolution medium, wherein the dissolution of the tested API can be further determined by a diffusion of the dissolved drug across the lipophilic membrane and by suitable establishing a suitable "driving force" through the membrane. Therefore, physiological conditions, that is the blood flow though the gut walls of the intestine, are reliably simulated. Thus, no unphysiological conditions are required which deteriorate the reliability of the prediction of in vivo conditions. Besides, the concept of the present invention provides for conductance of dissolution test with minimum amounts, for example as low volumes as 2.5 ml of test medium. Since such test media may comprise, for example very expensive compounds such as bile salts, significant cost reduction is provided by the present concept for testing dissolution.

The membrane may be a natural mammalian intestinal epithelial membrane which inherently comprises a lipophilic phase or which is first defatted by means known in the art, e.g. by applying appropriate defatting solvents, and then provided with a lipophilic phase by impregnating the membrane with said lipophilic phase By impregnation of the membrane with the lipophilic phase, it is possible that the surface of the membrane comprises said lipophilic phase, preferably the membrane surface and optionally the entire membrane surface is covered with said lipophilic phase, and the lipophilic phase is preferably also contained in the pores of the membrane.

The "lipophilic phase" may comprise, or may be solely constituted by, one or more lipophilic compounds. In case of a plurality of lipophilic compounds, these are preferably miscible such that one homogeneous lipophilic phase is formed, wherein said phase can be in fluid form or in solid form after impregnation. Appropriate lipophilic compounds are preferably selected from the group consisting of hydrocarbons, fatty acids, fatty alcohols and fatty acid esters of polyhydric alcohols. The different compounds for the lipophilic phase can be properly selected depending on the nature of the membrane. The membrane used in the present dissolution test may also be an artificial membrane made of an appropriate polymer material. For example, in the case of the above described example, an artificial cellulose nitrate membrane is used which is impregnated with 1-octanol as the lipophilic phase. However, other compounds or mixtures of compounds belonging to the same or different kinds of substance groups can be suitably selected as the lipophilic phase, and other polymer materials may be selected for the membrane material as well.

Brief description of the Drawing

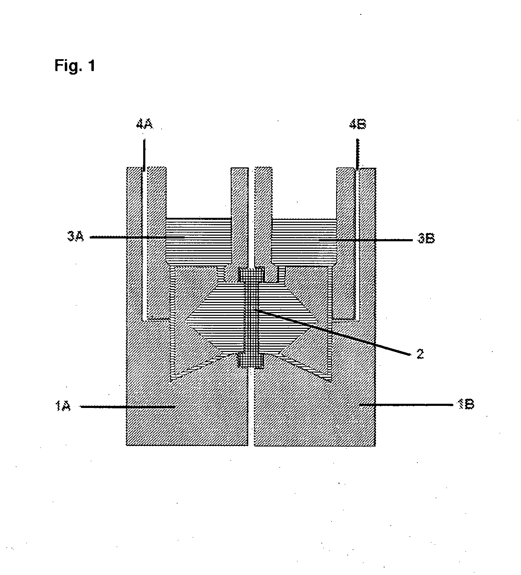

Fig. 1 shows a schematic drawing of a dissolution apparatus according to an embodiment of the invention.

Fig. 1 depicts the general constitution of the present apparatus for dissolution testing according to an embodiment of the present invention, which comprises two half-chambers (1A and 1 B) separated by a membrane (2) comprising a lipophilic phase. The formulation to be tested is placed in the dissolution medium in the donor compartment (3A). Dissolution medium in the donor compartment preferably reflects the *in vivo* conditions in the intestinal tract, regarding the ionic composition, concentration and type of surfactants, pH and temperature. An example for such a dissolution medium is Ringer buffer. The original Ringer buffer contains 140.6 $Na^+$, 5 $K^+$, 1.2 $Ca^{2+}$, 1.2 $Mg^{2+}$, 121.8 $Cl^-$, 25 $HCO_3^-$, 0.4 $H_2PO_4^-$, 1.6 $HPO_4^{2-}$ and has a pH 7.4 at 37 °C, when gassed with carbogen (mixture of 5% carbon dioxide and 95 % oxygen). The pH of Ringer buffer can also be set to any value between pH 6.0 and 7.5, which is the usual range in the intestinal tract, by adjusting the amount of $HCO_3^-$, $H_2PO_4^-$, $HPO_4^{2-}$. Additionally, the dissolution medium may contain bile salts in the concentration range (0.1-50 mM) and phospholipids (i.e. lecithin) in the concentration range (0.1-10 mM). Artificial membrane simulates the intestinal epithelial membrane. The artificial-membrane is permeable to dissolved tested API, but it is not permeable to undissolved tested API and to dissolution medium. The medium in the acceptor compartment (3B) does not necessarily reflect *in vivo* conditions. It is most important that the API, which is placed in the form of a pharmaceutical formulation in the donor compartment, has a high tendency to the acceptor medium, thus maintaining high concentration gradient across the artificial membrane, which simulates continuous wash away of the API by the intestinal blood flow. In order to generate a membrane permeation which is suitable for simulating said continuous wash away, an acceptor medium is selected in which the API is highly soluble. The higher solubility of the acceptor medium can be based on a simply higher solubility, preferably at least three times higher, of the API in the acceptor medium compared to the donor medium. Alternatively, higher solubility of the API in the acceptor medium compared to the donor medium is effected by the addition of substances that react with the API chemically, e.g. adjusting the pH appropriately, adding serum albumins etc., or physically. The media in the donor and acceptor compartments can optionally be mixed by applying gas mixture (i.e. carbogen) through the apertures (4A and 4B).

The following example is merely illustrative of the present invention and it should not be considered as limiting the scope of the invention in any way. The examples and modifications or other equivalents thereof will become apparent to those versed in the art in the light of the present entire disclosure.

<u>Example</u>

Two suspension formulations of the model drug, formulation A and formulation B, comprising 750 mg of the model drug per 5 ml formulation were tested separately in two-compartment dissolution apparatus according to the invention. Tests were performed 3-fold each for formulation A and formulation B, respectively. The measured data obtained from the three two-compartment dissolution apparatus testing the same formulation corresponded well with each other respectively and were averaged in order to obtain the measurement results indicated below. Accordingly, the reproducibility and the reliability of the apparatuses and the measurement have been verified. In the present example, said both suspension formulations exhibit a different extent of absorption in humans (AUC-A and AUC-B) under fasted state conditions. The model drug has an extremely low water solubility at pH values typical for the gastrointestinal tract, for example 0.03 $\mu$g/ml at pH 6.8, but relatively good solubility at very high pH values, e.g. 450 $\mu$g/ml at pH 12. The following procedure was used:

The artificial lipid barrier was prepared from a cellulose nitrate membrane filter with 0.1 $\mu$m pore size (Sartorius, Göttingen, Germany), which was impregnated with 1-octanol for 1 day. The excess of octanol was removed by pressing the impregnated membrane filter between the two pieces of blotting paper. Then, the impregnated filter disc was mounted between the two half-chambers. Afterwards, the membrane mounted into the apparatus was exposed to the same buffers as used later in the experiment for 60 min to ensure that the lipid phase was saturated with aqueous phase.

5 ml of the suspension to be tested was diluted to 250 ml of the donor dissolution medium. In the present example, Ringer buffer with pH 6.85 when gassed with carbogen, a mixture of 5% carbon dioxide and 95% oxygen, at 37°C, containing 3.85 mM sodium taurocholate and 1 mM lecithin was used as the donor dissolution medium. 2.5 ml of diluted suspension was placed in the donor compartment and 2.5 ml of 0.1 M sodium hydroxide was placed in the acceptor compartment. 1 ml of acceptor solution was taken every 90 minutes within a time range of 270 minutes and replaced by fresh 0.1 M NaOH. The concentration of the model drug in the acceptor compartment was analyzed by UV spectroscopy at 283 nm. The amount of API in the acceptor compartment at 270 minutes was calculated for formulation A and B and resulted in *in vitro* dissolution data indicated as $Q_{270-A}$ and $Q_{270-B}$. In $Q_{270-A}$ and $Q_{270-B}$, dilution due to the sampling every 90 min. was considered by calculating the Q-values according to the following formula:

$$Q_{270} = C_{270} \times V_{acceptor} + (V_{sample} \times C_{90} + V_{sample} \times C_{180})$$

$Q_{270}$:             amount of API (in grams) at 270 minutes
$V_{acceptor}$:             volume of the acceptor medium (i.e. 2.5 ml)
$V_{sample}$:             volume of the sample (i.e. 1 ml)
$C_{270}$; $C_{180}$; $C_{90}$:     concentration of API in the sample at a specific time (in this example: 90, 180 and 270 min.)

The person skilled in the art will readily understand how to modify the above formula in order to consider dilution in the case were the number of samples and/or the sampling times are modified compared to the above described example.

Table 1. Comparison of the ratio of *in vivo* pharmacokinetic data of formulation A to *in vivo* pharmacokinetic data of formulation B and the ratio of in vitro dissolution data of formulation A to in vitro dissolution data of formulation B.

| AUC-A / AUC-B | 0.79 |
|---|---|

(continued)

| Q$_{270-A}$ / Q$_{270-B}$ | 0.70 |
| --- | --- |

As evident from Table 1, a good in vitro-in vivo correlation was obtained for the tested formulations using the dissolution apparatus according to the present invention.

In the above example, dissolution testing of the model drug is described. As should become apparent, the present invention can be applied for dissolution testing of other APIs. When testing other APIs, an acceptor medium different from the described may be used, e.g. a relatively strong acidic aqueous media, a media comprising serum albumins or other means for chemically or physically improving solubility of the API to be tested. Generally, the composition of the acceptor medium is selected with the proviso that an effective driving force can be beneficially provided for simulating the continuous wash away of API by the intestinal blood flow. Furthermore, it is understood that the composition of the donor medium will depend on the intestine conditions, for example fasted state or fed state, to be tested.

## Claims

1.  A system for testing dissolution of an active pharmaceutical ingredient, which contains an apparatus comprising two compartments (3A, 3B) separated by a membrane (2), wherein the membrane (2) is permeable to a dissolved state but impermeable to an undissolved state of said active pharmaceutical ingredient, whereby the system comprises a donor medium placed in the first compartment representing the donor compartment (3A) and the system comprises
    an acceptor medium placed in the second compartment representing the acceptor compartment (3B)
    **characterised in that**
    said membrane (2) is impregnated with a lipophilic phase and that the active pharmaceutical ingredient is more soluble in the acceptor medium than in the donor medium or that acceptor medium is capable of reacting with the active pharmaceutical ingredient to thereby consume the active pharmaceutical ingredient by chemical reaction, so that a driving force is provided effecting that the active pharmaceutical ingredient passes the membrane (2) from the donor compartment (3A) to the acceptor compartment (3B).

2.  The system according to claim 1, wherein the apparatus is arranged such that when a donor medium comprising active pharmaceutical ingredient in undissolved state and optionally also in dissolved state is placed in said first compartment representing the donor compartment (3A), said second compartment representing the acceptor compartment (3B) comprises only active pharmaceutical ingredient in dissolved state in an acceptor medium.

3.  The system according to claim 1 or 2, further comprising the following means (a) and/or (b):

    (a) means for measuring and/or detecting the concentration of active pharmaceutical ingredient or a reaction or decomposition product thereof, is (are) arranged either in the acceptor compartment (3B), or in the donor compartment (3A), or both in the acceptor compartment (3B) and in the donor compartment (3A);
    (b) means for withdrawing sample(s) for subsequent measurement and/or detection of the concentration of active pharmaceutical ingredient or a reaction or decomposition product thereof, is (are) arranged either in the acceptor compartment (3B), or in the donor compartment (3A), or both in the acceptor compartment (3B) and in the donor compartment (3A).

4.  The system according to any one of claims 1 to 3, wherein the type of membrane (2) is selected from:

    (i) a membrane prior to impregnation with the lipophilic phase being an artificial membrane, and/or
    (ii) a membrane prior to impregnation with the lipophilic phase having an average pore size in a range of 0.1 μm to 10 μm.

5.  Process for testing dissolution of an active pharmaceutical ingredient, comprising the steps of:

    - providing a chamber comprising two compartments separated by a membrane (2) ,one compartment being a donor compartment (3A) donating the active pharmaceutical ingredient, and the other compartment being the acceptor compartment (3B) accepting or receiving the active pharmaceutical ingredient,
    - placing a donor medium comprising at least an active pharmaceutical ingredient and an aqueous solution in the donor compartment (3A),
- placing an acceptor medium in the acceptor compartment (3B), wherein the active pharmaceutical ingredient is allowed to pass the membrane (2) from the donor compartment (3A) to the acceptor compartment (3B), and
- measuring and/or detecting the concentration of active pharmaceutical ingredient or a reaction or decomposition product thereof, either in the acceptor medium, or measuring and/or detecting in the donor medium, or both in the acceptor medium and in the donor medium,

**characterised in that** said membrane is impregnated with a lipophilic phase, and further **characterised in that** a driving force between the donor compartment (3A) and the acceptor compartment (3B) is provided by selecting an acceptor medium in which the active pharmaceutical ingredient is more soluble than in the donor medium, or wherein the active pharmaceutical ingredient is consumed by chemical reaction.

6. The process according to claim 5, wherein one or more sample volume(s) of the acceptor medium in the acceptor compartment (3B) are taken every predetermined time interval(s), and said one or more sample volume(s) is (are) replaced by fresh acceptor medium after taking said sample volume(s).

7. The process according to any one of claims 5 or 6, wherein two or more varying conditions or forms of active pharmaceutical ingredient are separately processed and measured/detected in order to compare the amount of each of said active pharmaceutical ingredient in varying conditions or forms or a reaction or decomposition product thereof contained in the acceptor medium at a predetermined time interval.

8. A process for obtaining an active pharmaceutical ingredient with desired dissolution properties, comprising the steps of:

- providing multiple active pharmaceutical ingredients respectively varying in at least one condition or form,
- subjecting each active pharmaceutical ingredient of the predetermined condition or form to a process according to claim 5, and
- selecting the active pharmaceutical ingredient in predetermined condition or form which displays the desired dissolution properties.

9. The process according to claim 8, wherein the condition(s) of the active pharmaceutical ingredient are selected from (x) or/and (y):

(x) the condition of the active pharmaceutical ingredient is selected from particle size, polymorphic forms, bulk density and type of processing;
(y) the active pharmaceutical ingredient is incorporated into a pharmaceutical composition varying in drug load, type and amount of excipient, compression degree and type of processing.

10. The process according to claim 5, using a system according to claim 1 and/or wherein the membrane (2) is as defined in claim 4.


**Patentansprüche**

1. System zum Testen der Auflösung eines aktiven pharmazeutischen Bestandteils, welches einen Apparat enthält, welcher zwei durch eine Membran (2) getrennte Kammern (3A, 3B) umfasst, wobei die Membran (2) für einen aufgelösten Zustand des aktiven pharmazeutischen Bestandteils permeabel ist, aber für einen nicht aufgelösten Zustand nicht permeabel ist, wobei das System ein Donormedium umfasst, welches in der ersten, die Donorkammer (3A) darstellende Kammer eingebracht ist,
und das System
ein Empfängermedium umfasst, welches in der zweiten, die Empfängerkammer (3B) darstellende Kammer eingebracht ist, **dadurch gekennzeichnet, dass**
die Membran (2) mit einer lipophilen Phase imprägniert ist, und dass der aktive pharmazeutische Bestandteil im Empfängermedium löslicher als im Donormedium ist oder dass das Empfängermedium in der Lage ist, mit dem aktiven pharmazeutischen Bestandteil zu reagieren, um den aktiven pharmazeutischen Bestandteil durch chemische Reaktion zu verbrauchen, so dass eine treibende Kraft vorliegt, welche bewirkt, dass der aktive pharmazeutische Bestandteil durch die Membran (2) von der Donorkammer (3A) zu der Empfängerkammer (3B) hindurchgeht.

**2.** System gemäß Anspruch 1, wobei der Apparat so angeordnet ist, dass wenn ein Empfängermedium, welches aktiven pharmazeutischen Bestandteil in nicht aufgelöstem Zustand und wahlweise ebenso in aufgelöstem Zustand umfasst, in der ersten, die Donorkammer (3A) darstellende Kammer enthalten ist, wobei die zweite Kammer, welche die Empfängerkammer (3B) darstellt, nur aktiven pharmazeutischen Bestandteil in aufgelöstem Zustand in einem Empfängermedium umfasst.

**3.** System gemäß Anspruch 1 oder 2, weiterhin aufweisend die folgende Einrichtung(en) (a) und/oder (b):

(a) Einrichtung(en) zum Messen und/oder Detektieren der Konzentration von aktiven pharmazeutischen Bestandteil oder einem Reaktions- oder Zersetzungsprodukt davon, ist (sind) entweder in der Donorkammer (3A) oder in der Empfängerkammer (3B) oder sowohl in der Empfängerkammer (3B) als auch in der Donorkammer (3A) angeordnet;
(b) Einrichtung(en) zum Entnehmen von Probe(n) zur anschließenden Messung und/oder Bestimmung der Konzentration von aktivem pharmazeutischen Bestandteil oder einem Reaktions- oder Zersetzungsprodukt davon, ist (sind) entweder in der Empfängerkammer (3B) oder in der Donorkammer (3A) oder sowohl in der Empfängerkammer (3B) als auch in der Donorkammer (3A) angeordnet.

**4.** System gemäß einem der Ansprüche 1 bis 3, wobei der Typ der Membran (2) ausgewählt ist aus:

(i) einer Membran, die vor der Imprägnierung mit der lipophilen Phase eine künstliche Membran ist, und/oder
(ii) einer Membran, die vor der Imprägnierung mit der lipophilen Phase eine durchschnittliche Porengröße in einem Bereich von 0,1 $\mu$m bis 10 $\mu$m besitzt.

**5.** Verfahren zum Testen der Auflösung eines aktiven pharmazeutischen Bestandteils, umfassend die Schritte von:

- Bereitstellen eines Raumes, aufweisend zwei Kammern getrennt durch eine Membran (2), wobei eine Kammer eine Donorkammer (3A) ist, welche den aktiven pharmazeutischen Bestandteil abgibt, und die andere Kammer die Empfängerkammer (3B) ist, welche den aktiven pharmazeutischen Bestandteil aufnimmt oder empfängt,
- Einfüllen eines Donormediums, welches mindestens einen aktiven pharmazeutischen Bestandteil und eine wässrigen Lösung enthält, in die Donorkammer (3A),
- Einfüllen eines Empfängermediums in die Empfängerkammer (3B), wobei der aktive pharmazeutische Bestandteil durch die Membran (2) von der Donorkammer (3A) in die Empfängerkammer (3B) durchgehen kann, und
- Messen und/oder Bestimmen der Konzentration von aktivem pharmazeutischen Bestandteil oder eines Reaktions- oder Zersetzungsprodukts davon, entweder im Empfängermedium, oder Messen und/oder Bestimmen im Donormedium, oder sowohl im Empfängermedium als auch im Donormedium,

**dadurch gekennzeichnet, dass** die Membran mit einer lipophilen Phase imprägniert ist, und weiterhin **gekennzeichnet dadurch, dass** eine treibende Kraft zwischen der Donorkammer (3A) und der Empfängerkammer (3B) bereitgestellt wird durch Auswahl eines Empfängermediums, in welchem der aktive pharmazeutische Bestandteil löslicher ist als im Donormedium, oder wobei der aktive pharmazeutische Bestandteil durch chemische Reaktion verbraucht wird.

**6.** Verfahren gemäß Anspruch 5, wobei eine oder mehrere Probenvolumen (-volumina) des Empfängermediums in der Empfängerkammer (3B) zu jedem vorbestimmten Zeitintervall genommen wird, und diese(s) eine oder mehr Probenvolumen (-volumina) durch frisches Empfängermedium ersetzt wird (werden) nach dem Entnehmen des Probenvolumens/der Probenvolumina ersetzt wird / werden.

**7.** Verfahren gemäß einem der Ansprüche 5 oder 6, wobei zwei oder mehr variable Beschaffenheiten oder Formen eines aktiven pharmazeutischen Bestandteils unabhängig voneinander bearbeitet und gemessen/bestimmt werden, um die Menge von jedem des aktiven pharmazeutischen Bestandteils in variablen Beschaffenheiten oder Formen oder eines Reaktions- oder Zersetzungsprodukts davon, wie es im Empfängermedium enthalten ist, zu einem vorbestimmten Zeitintervall zu vergleichen.

**8.** Verfahren zum Erhalten eines aktiven pharmazeutischen Bestandteils mit gewünschten Auflösungseigenschaften, umfassend die Schritte von:

- Bereitstellen von mehreren aktiven pharmazeutischen Bestandteilen, sich jeweils unterscheidend in mindestens einer Beschaffenheit oder Form,
- Unterziehen von jedem der aktiven pharmazeutischen Bestandteilen der vorbestimmten Beschaffenheit oder Form einem Verfahren gemäß Anspruch 5, und
- Auswählen des aktiven pharmazeutischen Bestandteils in einer vorbestimmten Beschaffenheit oder Form, welche die gewünschten Auflösungseigenschaften zeigt.

9. Verfahren gemäß Anspruch 8, wobei die Beschaffenheit(en) des aktiven pharmazeutischen Bestandteils ausgewählt sind aus (x) oder/und (y):

(a) die Beschaffenheit des aktiven pharmazeutischen Bestandteils ist ausgewählt aus Partikelgröße, polymorphen Formen, Bulk-Dichte und des Typs der Verarbeitung;
(b) der aktive pharmazeutische Bestandteil wird in eine pharmazeutischen Zusammensetzung eingefügt, welche sich in der Medikamentenbeladung, des Typs und der Menge von Hilfsstoff, des Verpressungsgrades und des Typs der Verarbeitung unterscheidet.

10. Verfahren gemäß Anspruch 5 unter Verwendung eines Systems gemäß Anspruch 1 und/oder wobei die Membran (2) wie in Anspruch 4 definiert vorhanden ist.

## Revendications

1. Système pour tester une dissolution d'un ingrédient pharmaceutique actif, qui contient un appareil comprenant deux compartiments (3A, 3B) séparés par une membrane (2), dans lequel la membrane (2) est perméable à un état dissous mais imperméable à un état non dissous dudit ingrédient pharmaceutique actif, moyennant quoi le système comprend un milieu donneur placé dans le premier compartiment représentant le compartiment donneur (3A) et le système comprend
un milieu accepteur placé dans le deuxième compartiment représentant le compartiment accepteur (3B),
**caractérisé en ce que**
ladite membrane (2) est imprégnée d'une phase lipophile et **en ce que** l'ingrédient pharmaceutique actif est plus soluble dans le milieu accepteur que dans le milieu donneur ou **en ce que** le milieu accepteur est capable de réagir avec l'ingrédient pharmaceutique actif pour consommer ainsi l'ingrédient pharmaceutique actif par réaction chimique, de sorte qu'une force motrice soit fournie, permettant à l'ingrédient pharmaceutique actif de traverser la membrane (2) du compartiment donneur (3A) au compartiment accepteur (3B).

2. Système selon la revendication 1, dans lequel l'appareil est agencé de telle sorte que, lorsqu'un milieu donneur comprenant un ingrédient pharmaceutique actif à l'état non dissous et facultativement également à l'état dissous, est placé dans ledit premier compartiment représentant le compartiment donneur (3A), ledit deuxième compartiment représentant le compartiment accepteur (3B) comprend uniquement un ingrédient pharmaceutique actif à l'état dissous dans un milieu accepteur.

3. Système selon la revendication 1 ou 2, comprenant en outre les moyens (a) et/ou (b) suivants :

(a) un/des moyen(s) de mesure et/ou de détection de la concentration d'un ingrédient pharmaceutique actif ou d'un produit de la réaction ou de la décomposition de celui-ci est (sont) agencé(s) soit dans le compartiment accepteur (3B), soit dans le compartiment donneur (3A) soit à la fois dans le compartiment accepteur (3B) et dans le compartiment donneur (3A) ;
(b) un/des moyen(s) de retrait d'un/des échantillon(s) pour la mesure consécutive et/ou la détection de la concentration de l'ingrédient pharmaceutique actif ou d'un produit de la réaction ou de la décomposition de celui-ci, est (sont) agencé(s) soit dans le compartiment accepteur (3B), soit dans le compartiment donneur (3A), soit à la fois dans le compartiment accepteur (3B) et dans le compartiment donneur (3A).

4. Système selon l'une quelconque des revendications 1 à 3, dans lequel le type de membrane (2) est choisi parmi :

(i) une membrane avant l'imprégnation avec la phase lipophile qui est une membrane artificielle, et/ou
(ii) une membrane avant l'imprégnation avec la phase lipophile ayant une taille de pore moyenne dans la plage de 0,1 μm à 10 μm.

5. Procédé pour tester une dissolution d'un ingrédient pharmaceutique actif, comprenant les étapes de :

- fourniture d'une chambre comprenant deux compartiments séparés par une membrane (2), un compartiment étant un compartiment donneur (3A) donnant l'ingrédient pharmaceutique actif, et l'autre compartiment étant le compartiment accepteur (3B) acceptant ou recevant l'ingrédient pharmaceutique actif,
- placement d'un milieu donneur comprenant au moins un ingrédient pharmaceutique actif et une solution aqueuse dans le compartiment donneur (3A),
- placement d'un milieu accepteur dans le compartiment accepteur (3B), dans lequel l'ingrédient pharmaceutique actif peut traverser la membrane (2) du compartiment donneur (3A) au compartiment accepteur (3B), et
- mesure et/ou détection de la concentration d'un ingrédient pharmaceutique actif ou d'un produit de la réaction ou de la décomposition de celui-ci, dans le milieu accepteur, ou mesure et/ou détection dans le milieu donneur, à la fois dans le milieu accepteur et dans le milieu donneur,

**caractérisé en ce que** ladite membrane est imprégnée d'une phase lipophile, et **caractérisé en outre en ce qu'**une force motrice entre le compartiment donneur (3A) et le compartiment accepteur (3B) est fournie en sélectionnant un milieu accepteur dans lequel l'ingrédient pharmaceutique actif est plus soluble que dans le milieu donneur, ou dans lequel l'ingrédient pharmaceutique actif est consommé par la réaction chimique.

6. Procédé selon la revendication 5, dans lequel un ou plusieurs volume(s) d'échantillon du milieu accepteur dans le compartiment accepteur (3B) sont prélevés à tout intervalle/tous les intervalles prédéterminé(s), et ledit/lesdits un ou plusieurs volume(s) d'échantillon est/sont remplacé(s) par un milieu accepteur frais après prélèvement dudit/desdits volume(s) d'échantillon.

7. Procédé selon l'une quelconque des revendications 5 ou 6, dans lequel deux ou plusieurs conditions ou formes variables d'ingrédient pharmaceutique actif sont traitées et mesurées/détectées séparément afin de comparer la quantité de chacun desdits ingrédients pharmaceutiques actifs dans des conditions ou formes variables ou un produit de la réaction ou de la décomposition de celui-ci contenu dans le milieu accepteur à un intervalle temporel prédéterminé.

8. Procédé d'obtention d'un ingrédient pharmaceutique actif présentant des propriétés de dissolution souhaitées, comprenant les étapes de :

- fourniture de multiples ingrédients pharmaceutiques actifs variables respectivement dans au moins une condition ou forme,
- soumission de chaque ingrédient pharmaceutique actif de la condition ou forme prédéterminée à un procédé selon la revendication 5, et
- sélection de l'ingrédient pharmaceutique actif dans une condition ou forme prédéterminée, qui présente les propriétés de dissolution souhaitées.

9. Procédé selon la revendication 8, dans lequel la/les condition(s) de l'ingrédient pharmaceutique actif sont choisies parmi (x) et/ou (y) ;

(x) la condition de l'ingrédient pharmaceutique actif est choisie parmi une taille de particule, des formes polymorphiques, une densité apparente et un type de traitement ;
(y) l'ingrédient pharmaceutique actif est incorporé dans une composition pharmaceutique variable en termes de charge médicamenteuse, de type et de quantité d'excipient, de degré de compression et de type de traitement.

10. Procédé selon la revendication 5, utilisant un système selon la revendication 1 et/ou dans lequel la membrane (2) est telle que définie dans la revendication 4.

**Fig. 1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030088369 A1
- WO 2008112245 A
- WO 9928437 A

**Non-patent literature cited in the description**

- **E. NICOLAIDES et al.** *Pharmaceutical Research,* 1999, vol. 16 (12), 1876-1882
- **J.B. DRESSMAN et al.** *European Journal of Pharmaceutical Sciences,* 2000, vol. 11 (2), 73-80
- **V.H. SUNESEN et al.** *European Journal of Pharmaceutical Sciences,* 2005, vol. 24, 305-313
- **G. CORTI et al.** Development and evaluation of an in vitro method for prediction of human drug absorbtion - I. Assessment of artificial membrane composition. *European Journal of Pharmaceutical Sciences,* 2005, vol. 27, 346-353
- Permeability through DOPC/dodecane membranes: measurement and LFER modelling. **HUQUE F.T.T. et al.** European Journal of Pharmaceutical Sciences. Elsevier, 2004, vol. 23, 223-232